Europäisches Patentamt

(19) European Patent Office

Office européen des brevets

(11) Numéro de publication : **0 445 477 A2**

# (12) DEMANDE DE BREVET EUROPEEN

(21) Numéro de dépôt : **90403333.9**

(22) Date de dépôt : **26.11.90**

(51) Int. Cl.$^5$ : **G01N 21/85, A61M 1/36, G01N 21/53**

(30) Priorité : **01.12.89 FR 8915855**

(43) Date de publication de la demande :
**11.09.91 Bulletin 91/37**

(84) Etats contractants désignés :
**DE GB IT**

(71) Demandeur : **ETS. F. ESPA ET CIE**
**Z.I. rue de l'Etain, B.P. 19**
**F-77541 Savigny le Temple (FR)**

(72) Inventeur : **Magnard, A.**
**29 bis rue de la Gueyere**
**F-91310 Longpont Sur Orge (FR)**
Inventeur : **Thomas, Ph.**
**12 rue des Troenes Rés. du Parc Les Charmilles**
**F-91700 Villiers/Orge (FR)**
Inventeur : **Thepaut, D.**
**9 Route de Sandreville**
**F-91580 Villeconin (FR)**

(54) **Procédé et dispositif de détection de bulles dans un fluide liquide et appareil les mettant en oeuvre.**

(57)     L'invention concerne un dispositif de détection de bulles dans un fluide liquide.

Le dispositif de détection selon l'invention comporte un support 11 une tubulure d'alimentation 7, une source lumineuse à faisceaux parallèles 15, une caméra 16 à réseau de transfert de charge, à laquelle est intégrée une unité de transformation des signaux lumineux en signaux électriques analogiques, une unité d'analyse 17 de ces signaux et une unité de commande 18 constituée d'un microprocesseur et d'un logiciel qui délivrera un signal d'arrêt de la pompe 8 dès qu'une bulle sera détectée.

Application à la détection de bulles dans un fluide liquide pour les domaines du médical, de la carburation, de l'hydraulique.

EP 0 445 477 A2

# PROCEDE ET DISPOSITIF DE DETECTION DE BULLES DANS UN FLUIDE LIQUIDE ET APPAREIL LES METTANT EN OEUVRE

L'invention concerne un procédé de détection de bulles ainsi qu'un dispositif de mise en oeuvre utilisé notamment dans le domaine médical mais pouvant également être utilisé dans des circuits de carburant ou hydrauliques.

Dans le domaine médical ou vétérinaire, les moyens destinés à administrer à des patients une ou des solutions comme par exemple du sang ou du glucose peuvent se faire soit en mode perfusion dans les services d'anesthésie ou dans des laboratoires de recherche, soit en mode accélération dans des services de réanimation d'urgence et de traumatologie. Aujourd'hui encore un appareil différent est utilisé selon le mode de transfusion. De plus, les procédés utilisés jusqu'à présent ne permettent pas d'identifier avec précision l'état du liquide qui circule dans le tube d'alimentation avant d'être administré au patient. Il est en effet essentiel de détecter la présence de bulles d'air dans le liquide car, dans le cas d'injection de sang par exemple, on sait que l'oxygène contenu dans les bulles d'air crée une oxydation du sang générant des micros-emboles ou caillots pouvant entrainer une embolie du type pulmonaire, cérébrale, cardiaque ou autre. Si dans des applications en type veineux des micros-emboles peuvent être dans certains cas acceptables mais pas recommandés, en type artériel ils peuvent avoir de très graves conséquences pouvant aller jusqu'au décès du patient. Il est donc extrèmement important, que se soit en mode perfusion ou en mode accélération, de détecter efficacement les bulles d'air se trouvant dans la tubulure d'alimentation et ce, quel que soit le débit et la nature du liquide à administrer.

Les dispositifs utilisés jusqu'à présent comportent un réservoir contenant le liquide à administrer qui est conduit, par l'intermédiaire d'une tubulure souple, à une pompe, en général du type péristaltique. Le débit du liquide contenu dans la tubulure est réglé par la pompe. Le liquide circulant dans la tubulure est contrôlé par un détecteur. Le liquide contenu dans la tubulure s'écoule dans le bras du patient par l'intermédiaire d'un cathéter implanté soit dans une veine soit dans une artère.

Les systèmes de détection de bulles connus aujourd'hui sont de plusieurs types:

– Les détecteurs de type capacitif utilisent une capacité qui mesure en permanence un champ électrique. Le changement d'état du liquide circulant dans la tubulure d'alimentation dû à la présence de bulles crée une modification du champ électrique qui se transforme en un signal entraînant l'arrêt de la pompe.

– Les détecteurs de type magnétique fonctionnent de la même manière que les détecteurs capacitifs, mais mesurent dans cette configuration un champ magnétique au lieu d'un champ électrique.

– Les détecteurs de type optique utilisent en général une cellule à infrarouge qui analyse le liquide qui s'écoule dans la tubulure d'alimentation. Lorsqu'une bulle passe devant ce détecteur il y a un front de changement de luminosité qui intervient et qui fait déclencher la pompe.

Les inconvénients de ces systèmes se situent au niveau de la reconnaissance des liquides employés. En effet, ces systèmes n'arrivent pas à distinguer les liquides clairs des liquides foncés et ils font déclencher la pompe intempestivement. Ces arrêts sont préjudiciables car en cours de perfusion ou de transfusion accélérée le praticien peut être amené à administrer au patient successivement des liquides de couleur et/ou de densité différentes.

L'invention s'affranchit des difficultés indiquées ci-avant et a pour objet un procédé de détection du passage de bulles d'air dans le flux d'un liquide en déplacement dans une tubulure transparente, rectiligne, caractérisé en ce que:

– on éclaire transversalement la tubulure contenant le flux de liquide au moyen d'une source de lumière à faisceaux parallèles;

– on transforme les signaux lumineux en signaux électriques analogiques représentatifs de la tache lumineuse reçue par une caméra à réseau de transfert de charges;

– on analyse la répartition des dits signaux sur la surface de détection de la caméra, ladite répartition étant fonction des modifications de chemins optiques à l'intérieur du flux de liquide lors de la rencontre des rayons lumineux avec les parois d'une bulle d'air circulant dans le flux;

– on compare le signal fournit à des signaux de référence mémorisés, significatifs l'un d'un trajet optique non modifié correspondant à une absence de bulle dans le flux, l'autre d'un trajet optique modifié par la présence d'une bulle dans le flux;

– on utilise ledit signal reçu et ainsi comparé pour commander une unité de commande apte à déclencher un processus tel que par exemple l'interruption du débit de fluide lorsqu'une bulle a été détectée.

L'invention a également pour objet un dispositif d'application du procédé comportant un support de tubulure déterminant une section rectiligne de tubulure, une source de lumière illuminant ladite section transversalement, une caméra disposée symétriquement à la source de lumière par rapport à l'axe de ladite section pour percevoir les faisceaux de lumière émis par la source et ayant traversée ladite section contenant le débit de fluide à analyser, une unité de transformation du signal lumineux en signal électrique analogique, une unité de calcul apte à comparer ledit signal électrique à des signaux de référence mémorisés, et une unité de commande apte à déclencher un processus lorsque ledit signal électrique est conforme à un signal de référence représentatif du passage d'une bulle d'air dans le débit de fluide.

L'invention a pour autre objet un dispositif dont la source lumineuse permettant de créer l'image du flux de liquide dans le conduit est à faisceaux parallèles, le spectre d'émission pouvant aller de l'infrarouge à l'ultraviolet.

L'invention a encore pour objet un dispositif dont la source lumineuse est soit une lampe à incandescence, soit une diode électro-luminescente du type LED, soit un émetteur laser.

L'invention a aussi pour objet un dispositif dont la caméra et l'unité de transformation des signaux lumineux sont constitués par une caméra à réseau de transfert de charges à structure soit linéaire soit matricielle du type CCD.

L'invention a aussi pour objet un dispositif dont la caméra comporte une optique de focalisation de l'image réalisée soit par la paroi de la tubulure ellemême soit par des lentilles appropriées.

L'invention a aussi pour objet un dispositif dont le support de tubulure est constitué d'un bâti en alliage léger ou en matière plastique muni d'une gorge rectiligne possèdant une découpe parallèle à l'axe de la gorge et de largeur légèrement inférieure au diamètre de la gorge, apte à recevoir la tubulure.

L'invention a aussi pour objet un dispositif dont le support de tubulure comporte un détecteur de positionnement de la tubulure dans la gorge, ce détecteur pouvant être soit à infrarouge soit à micro-contact.

L'invention a encore pour objet un dispositif de détection de bulles dans un fluide incorporé à un appareil pouvant fonctionner soit en mode perfuseur soit en mode accélérateur comportant au moins une pompe apte à faire circuler le fluide, une unité de commande apte à déclencher un processus tel que la commande d'une alarme, la commande de l'interruption de la pompe, la commande d'un réchauffeur de sang.

L'invention a aussi pour objet un dispositif de détection de bulles situé en aval de la pompe.

L'invention sera décrite plus en détail en regard des dessins annexés à titre nullement limitatif et sur lesquels:

    – la figure 1 est une vue de face d'un appareil perfuseur/accélérateur équipé d'un détecteur de bulles selon l'invention.

    – la figure 2 est une vue de dessus du support du détecteur de bulles selon l'invention sans que la tubulure soit représentée.

    – La figure 3 est une vue selon la coupe AA de la figure 2.

    – La figure 4 est un shéma de principe de fonctionnement d'un détecteur de bulles selon l'invention.

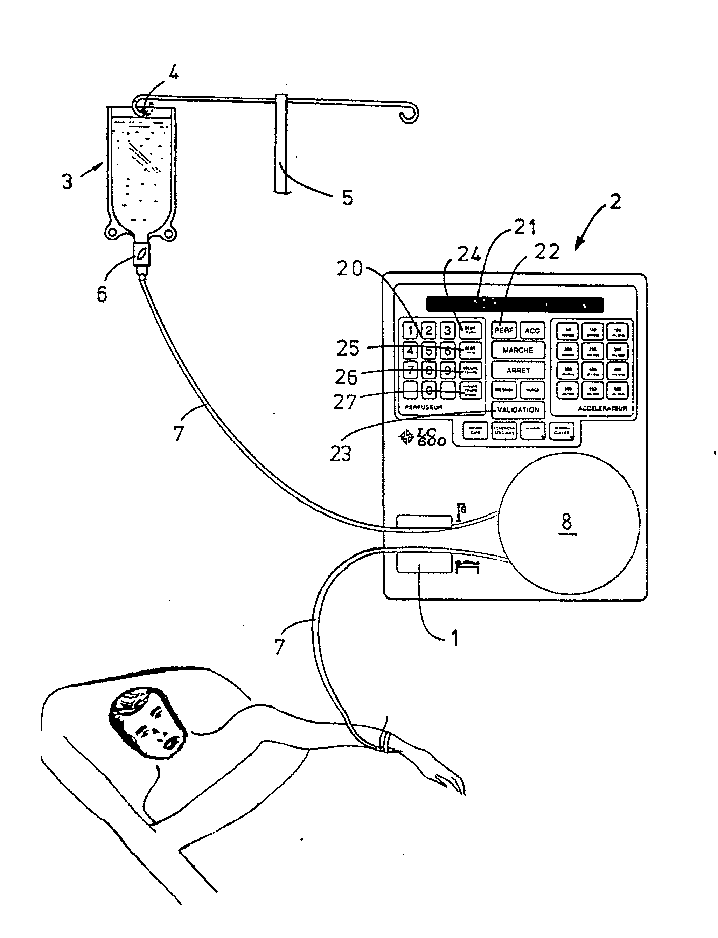

Dans le mode de réalisation représenté à la figure 1 le dispositif de détection de bulles 1 selon l'invention est intégré sur la partie inférieure gauche d'un appareil 2 pouvant fonctionner en mode perfuseur ou en mode accélérateur de transfusion.

Le liquide devant être administré au patient est contenu dans un récipient 3 généralement transparent comportant à sa partie supérieure un moyen 4 permettant de le suspendre à un support 5 et à sa partie inférieure un embout 6 permettant le raccordement d'une tubulure d'alimentation 7. Cette tubulure d'alimentation 7 qui est flexible et transparente relie le récipient 3 au patient en passant préalablement tout d'abord dans une pompe 8 de type péristaltique à trois galets qui règle le débit, puis devant le détecteur de bulles 1.

Pour alimenter le patient le praticien met l'appareil 2 sous tension en appuyant sur un interrupteur situé sur la face arrière de l'appareil 2 ce qui entraîne immédiatement l'affichage sur l'écran 21 de l'appareil 2, sous forme de question, le choix du mode à utiliser, à savoir soit perfuseur soit accélérateur.

Il sera décrit ci-après une utilisation en mode perfuseur. Le praticien sélectionne par la touche 22 le mode perfusion et validera par la touche 23 ce choix, à ce moment apparaîtra sur l'écran 21 toujours sous forme de question le choix du mode de débit. Celui-ci peut être de quatre modes différents. En effet, le praticien pourra en fonction de l'état du patient par le clavier 20 régler la valeur du débit de liquide d'alimentation soit en ml/mn par la touche 24, soit en ml/H par la touche 25, soit en demandant par la touche 26 au logiciel de l'unité de commande 18 de calculer le débit en entrant les données par le clavier 20 relatives au volume à administrer dans un temps déterminé, soit en demandant par la touche 27 au logiciel de l'unité de commande 18 de calculer le débit du liquide d'alimentation en lui entrant par le clavier 20 les données relatives au volume à administrer dans un temps déterminé en tenant compte du poids du patient.

La touche marche lancera alors le moteur pas à pas 19 de la pompe 8 et le liquide se trouvant dans la tubulure 7 s'écoulera. Le liquide passera pour être analysé devant le détecteur de bulles 1 se trouvant en aval de la pompe 8.

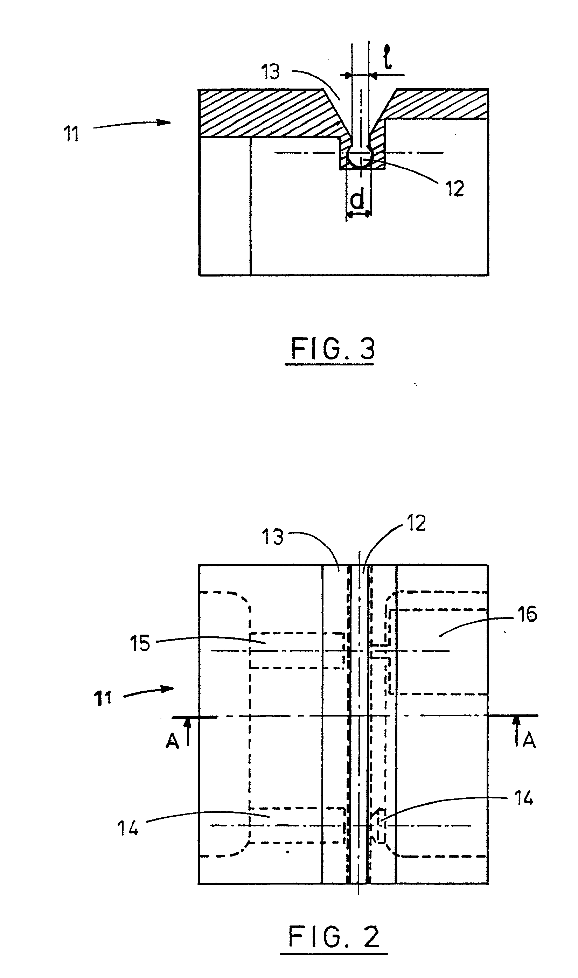

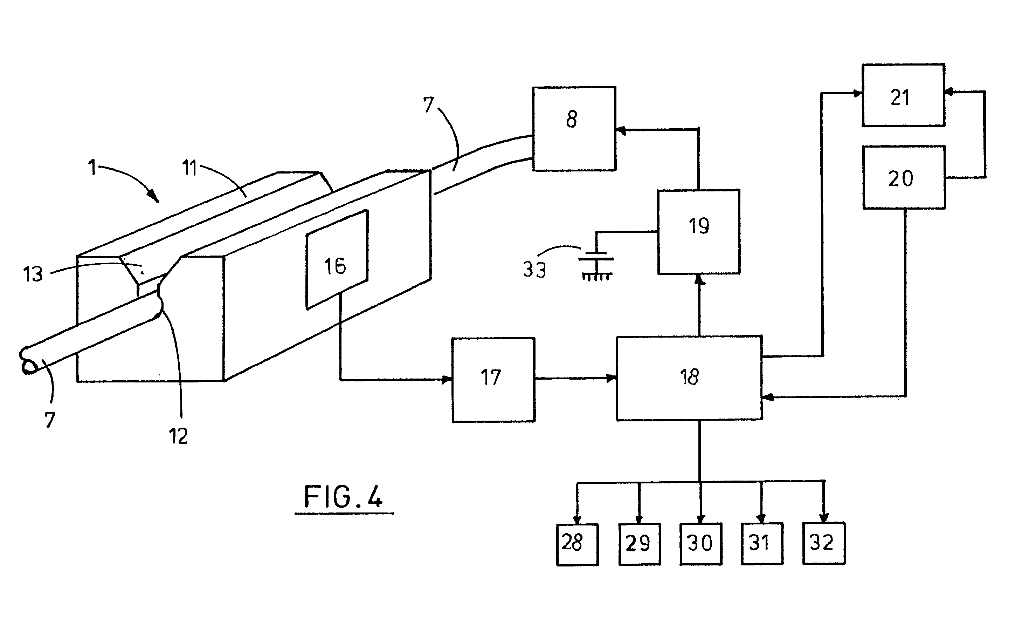

La description du dispositif de détection de bulles 1 conforme à l'invention sera mieux comprise en regard des figures 2, 3 et 4.

Le détecteur de bulles 1 est constitué d'un support rectiligne 11 en alliage léger ou en matière plastique muni d'une gorge 12 rectiligne possèdant une découpe 13 parallèle à l'axe de la gorge 12 et de largeur 1 légèrement inférieure au diamètre d de la gorge 12 apte à recevoir la tubulure d'alimentation 7. Une source lumineuse 15 est placée sur le support 11 perpendiculairement à l'axe de la gorge 12 illuminant ainsi une section transversale de la tubulure d'alimentation 7. Cette source lumineuse 15 permettant de créer l'image du flux de liquide circulant dans la tubulure d'alimentation 7 est à faisceaux parallèles, le spectre d'émission pouvant aller de l'infrarouge à l'ultraviolet. Cette source lumineuse 15 pourra être avantageusement soit une lampe à incandescence soit une diode électro-luminescente du type LED soit un émetteur laser. La caméra et son unité de transformation de signaux lumineux sont constitués par une caméra 16 à réseau de transfert de charges à structure soit matricielle soit linéaire.

La caméra 16 est disposée symétriquement à la source de lumière 15 pour percevoir les faisceaux de lumière ayant traversé la section de la tubulure d'alimentation 7 contenant le débit de fluide à analyser. L'unité de transformation intégrée à la caméra 16 transformera les signaux lumineux reçus en signaux électriques analogiques qui seront transmis à un analyseur d'images 17 qui analysera la répartition desdits signaux captés par la surface de détection de la caméra 16. La répartition étant fonction des modifications des chemins optiques à l'intérieur du flux de liquide lors de la rencontre des rayons lumineux avec les parois d'une bulle d'air circulant dans le liquide d'alimentation.

En effet, lorsque les rayons lumineux rencontrent les parois d'une bulle d'air on remarque alors que les rayons ne sont plus parallèles car il y a une diffraction de ceux-ci. Les signaux analysés sont dirigés vers une unité de commande 18 constituée par un microprocesseur et un logiciel. Cette unité de commande 18 comparera les signaux reçus à des signaux de référence mémorisés, significatifs l'un d'un trajet optique non modifié correspondant à l'absence de bulle dans le flux, l'autre d'un trajet optique modifié par la présence d'une bulle dans le flux du liquide d'alimentation.

En fonction du type de signaux reçus l'unité de commande 18 commandera l'arrêt ou non du moteur pas à pas 19 qui entraîne la pompe 8 en rotation déclenchant une alarme sonore 29 interne à l'appareil ou externe ainsi qu'une alarme visuelle constituée d'un message sur l'écran 21 de l'appareil 2.

Afin d'obtenir une souplesse supplémentaire d'utilisation, les informations mémorisées dans l'unité de commande 18 permettent, lorsque dans une séquence d'alimentation, la première bulle est détectée, de donner au praticien le choix, soit de laisser passer la bulle si celle-ci ne lui semble pas dangereuse pour le patient, soit de mettre en oeuvre le processus de purge.

L'optique de focalisation de la caméra pourra être avantageusement soit la paroi de la tubulure d'alimentation 7 elle-même, soit des lentilles appropriées.

Le support 11 comporte également à une de ses extrêmités un détecteur 14 de positionnement de la tubulure d'alimentation 7.

Ce détecteur 14 de positionnement vérifiera si la tubulure d'alimentation 7 est bien positionnée dans le fond de la gorge 12 ce qui implique d'une part que la tubulure d'alimentation 7 est bien rectiligne et que d'autre part, la tubulure d'alimentation 7 se trouve bien en position devant la caméra 16.

Ce détecteur 14 qui est disposé perpendiculairement à la gorge 12 peut être soit à infrarouge soit à micro-contact. En cas de mauvais positionnement de la tubulure d'alimentation 7 le détecteur 14 délivrera des signaux à l'unité de commande 18 tels que celle-ci interdira d'une part la mise en fonctionnement du moteur de la pompe et d'autre part déclenchera une alarme sonore 29 interne à l'appareil ou externe à celui-ci ainsi qu'une alarme visuelle constituée par un message sur l'écran 21 de l'appareil 2.

L'appareil comportant le dispositif de détection de bulles selon l'invention a pour autres avantages le fait qu'il soit léger, transportable pouvant fonctionner sous toutes les tensions existantes aujourd'hui et également en mode interne par l'intermédiaire d'une batterie 33 de 2 heures d'autonomie. Les informations mémorisées dans l'unité de commande 18 permettent de commander un réchauffeur de sang 31, des dispositifs d'asservissements 28, des alarmes 29 de toutes sortes, mais également d'effectuer de la télésurveillance 32 à domicile ou d'être raccordé à un ordinateur central 30.

Bien que l'invention ait été décrite dans son application à un dispositif de détection de bulles dans un fluide liquide à des fins médicales, il est évident que ce dispositif peut être utilisé pour la détection de bulles à des fins autres que médicales, comme par exemple la détection de bulles d'air dans des circuits de carburant notamment avant le carburateur. Il peut également être très important de détecter des bulles d'air circulant dans un fluide hydraulique, quand on sait les conséquences importantes que peut déclencher une bulle d'air dans le circuit hydraulique de commande de freinage d'un véhicule.

Il va de soi que de nombreuses modifications peuvent être apportées au dispositif décrit sans pour autant sortir du cadre de l'invention.

## Revendications

1. Procédé de détection du passage de bulles d'air dans le flux d'un liquide en déplacement dans une tubulure transparente rectiligne caractérisé en ce que:

   – on éclaire transversalement la tubulure d'alimentation 7 contenant le flux de liquide au moyen d'une source de lumière 15 à faisceaux parallèles,

– on enregistre au moyen d'une caméra 16 l'image du faisceau ayant traversé la tubulure d'alimentation 7 et le flux de liquide,

– on transforme les signaux lumineux en signaux électriques analogiques représentatifs de la tache lumineuse reçue par la caméra 16,

– on analyse la répartition desdits signaux sur la surface de détection de la caméra 16, ladite répartition étant fonction des modifications de chemins optiques à l'intérieur du flux de liquide lors de la rencontre des rayons lumineux avec les parois d'une bulle d'air circulant dans le flux,

– on compare le signal fourni à des signaux de référence mémorisés, significatifs l'un d'un trajet optique non modifié correspondant à une absence de bulle dans le flux, l'autre d'un trajet optique modifié par la présence d'une bulle dans le flux,

– on utilise ledit signal reçu et ainsi comparé pour commander une unité de commande 18 apte à déclencher un processus tel que par exemple l'interruption du débit de fluide lorsqu'une bulle a été détectée.

2. Dispositif d'application du procédé selon la revendication 1 caractérisé en ce qu'il comporte en combinaison un suport de tubulure 11 déterminant une section rectiligne de tubulure 7, une source de lumière 15 à faisceaux parallèles illuminant ladite section transversalement, une caméra 16 disposée symétriquement à la source de lumière 15 par rapport à l'axe de ladite section pour percevoir les faisceaux de lumière émis par la source et ayant traversée ladite section contenant le débit de fluide à analyser, une unité de transformation 16 du signal lumineux en signal électrique analogique, une unité d'analyse 17 de la répartition dudit signal sur la surface de détection de la caméra 16, une unité de calcul 18 apte à comparer ledit signal électrique à des signaux de référence mémorisés, et une unité de commande 18 apte à déclencher un processus lorsque ledit signal est conforme à un signal de référence représentatif du passage d'une bulle d'air dans le débit de fluide.

3. Dispositif selon la revendication 2 caractérisé en ce que la source de lumière 15 est un émetteur laser.

4. Dispositif selon la revendication 2 caractérisé en ce que la source de lumière est une diode électro-luminescente.

5. Dispositif selon l'une des revendications 2 à 4 caractérisé en ce que la caméra et l'unité de transformation 16 des signaux lumineux sont constitués par une caméra 16 à structure linéaire du type à transfert de charges dite CCD.

6. Dispositif selon l'une des revendications 2 à 4 caractérisé en ce que la caméra et l'unité de transformation 16 des signaux lumineux sont constitués par une caméra 16 à structure matricielle du type à transfert de charge dite CCD.

7. Dispositif selon la revendication 6 caractérisé en ce que la caméra 16 comporte une optique de focalisation.

8. Dispositif selon la revendication 7 caractérisé en ce que l'optique de focalisation est constituée par la paroi de la tubulure 7 elle-même.

9. Dispositif selon la revendication 7 caractérisé en ce que l'optique de focalisation est constituée par des lentilles appropriées.

10. Dispositif selon l'une quelconque des revendications 2 à 6 caractérisé en ce que le support 11 de tubulure 7 est constitué d'un bâti muni d'une gorge 12 rectiligne possèdant une découpe d'introduction 13 parallèle à l'axe de la gorge 12 et de largeur l légèrement inférieure au diamètre d de la gorge 12, apte à recevoir la tubulure 7.

11. Dispositif selon la revendication 10 caractérisé en ce que le support 11 de tubulure 7 est équipé d'un détecteur 14 de positionnement de la tubulure 7 dans la gorge 12.

12. Dispositif selon la revendication 11 caractérisé en ce que le détecteur 14 de positionnement de la tubulure 7 est à infrarouge.

13. Dispositif de détection de bulles 1 dans un fluide selon l'une quelconque des revendications 2 à 12 caractérisé en ce qu'il est incorporé à un appareil perfuseur accélérateur 2 comportant au moins une pompe 8 apte à faire circuler un fluide depuis un récipient 3 vers un cathéter placé dans la veine du malade, une unité de commande 18 apte à déclencher un processus tel que la commande d'une alarme 29, la commande de l'interruption de la pompe 8.

14. Dispositif selon la revendication 13 caractérisé en ce que le dispositif de détection de bulles 1 est situé en aval de la pompe 8.

15. Dispositif selon les revendications 13 et 14 caractérisé en ce que la pompe 8 du type péristaltique à trois galets est commandée par un moteur pas à pas recevant son signal de commande de l'unité de commande 18.

FIG. 3

FIG. 2

FIG.4

EP 0 445 477 A2